# EUROPÄISCHE PATENTSCHRIFT

(11) **EP 0 760 640 B1**
(45) Veröffentlichungstag und Bekanntmachung des Hinweises auf die Patenterteilung: **26.08.1998**
(21) Anmeldenummer: 95917975.5
(22) Anmeldetag: 27.04.1995
(51) Int. Cl.: A61F 2/76

(54) **TESTPROTHESE**
TEST PROSTHESIS
PROTHESE D'ESSAI

(30) Priorität: 25.05.1994 DE 9408556 U
(43) Veröffentlichungstag der Anmeldung: 12.03.1997
(73) Patentinhaber: BIEDERMANN MOTECH GmbH, 78054 Villingen-Schwenningen (DE)
(72) Erfinder: BIEDERMANN, Lutz, D-78048 VS-Villingen (DE)
(74) Vertreter: Prüfer, Lutz H., Dipl.-Phys.
(86) Internationale Anmeldenummer: EP9501609
(87) Internationale Veröffentlichungsnummer: WO9531949

(56) Entgegenhaltungen:
- EP-A- 0 439 028
- GB-C- 1 114 312
- US-A- 3 538 516
- US-A- 3 551 915

## Beschreibung

Die Erfindung betrifft eine Testprothese nach dem Oberbegriff des Patentanspruches 1.

Eine Prothese für einen Oberschenkelamputierten, d.h. für eine Person, bei der eine Amputation des Beines oberhalb des Knies vorgenommen wurde, weist herkömmlicherweise ein Oberschenkelteil, ein Unterschenkelteil mit einem Fußteil, ein Gelenk und eine Schwungphasensteuerung auf, wobei das Oberschenkelteil und das Unterschenkelteil über das Gelenk schwenkbar miteinander verbunden sind. Die Schwungphasensteuerung weist ein erstes und ein zweites Widerlager auf, wobei das erste Widerlager mit dem Oberschenkelteil und das zweite Widerlager mit dem Unterschenkelteil verbunden sind.

Für solche Arten von Prothesen gibt es die verschiedensten Arten von Unterschenkelteilen, Fußteilen, Schwungphasensteuerungen, Standphasensicherungen, etc., die sich erstens in ihren Eigenschaften, die an die Bedürfnisse des Oberschenkelamputierten angepaßt werden müssen, und zweitens in ihren Preisen unterscheiden. Die Anatomie verschiedener Personen ist unterschiedlich, und daher ist eine Prothese für einen Oberschenkelamputierten zusätzlich an die entsprechende Anatomie anzupassen. Für die Auswahl der verschiedenen Komponenten einer solchen Prothese und für die Anpassung an die Bedürfnisse und die Anatomie des Oberschenkelamputierten ist es notwendig, verschiedene Kombinationen der möglichen Komponenten und deren Anpassung an die Anatomie des Oberschenkelamputierten ausprobieren zu können.

Dabei ergibt sich z.B. das Problem, daß die unterschiedlichen Schwungphasensteuerungseinrichtungen und/oder Standphasensicherungseinrichtungen unterschiedliche Baugrößen und/oder Verbindungsmöglichkeiten und -anforderungen aufweisen, daß die unterschiedlichen unterschenkelteile und Füße schon aufgrund ihrer unterschiedlichen Eigenschaften sehr unterschiedlich ausgebildet sind, und daß daher der Austausch verschiedener Komponenten bei einer Prothese oft gar nicht oder nur mit sehr großem Aufwand möglich ist. Die Auswahl der richtigen Prothese mit dem dazu notwendigen Ausprobieren verschiedener Komponenten ist daher sehr schwierig und aufwendig.

Aus der EP-A-0 439 028 ist eine Testprothese nach dem Oberbegriff des Patentanspruches 1 bekannt. Aus der US-A-3 538 516 ist eine Prothese mit einem Oberschenkelteil, einem Unterschenkelteil und einer das Oberschenkelteil mit dem Unterschenkelteil verbindenden Verbindungseinheit bekannt, wobei die Verbindungseinheit eine Relativbewegung zwischen den verbundenen Teilen in einer zur Beinachse geneigten Richtung ermöglicht.

Es ist Aufgabe der vorliegenden Erfindung, eine Testprothese zu schaffen, mit der die Kombination verschiedener Komponenten einer Prothese einfach möglich ist.

Diese Aufgabe wird gelöst durch eine Testprothese nach Anspruch 1.

Weiterbildungen der Erfindung sind in den Unteransprüchen gekennzeichnet.

Die Ausbildung des Knieabschnitts einer Ausführungsform ermöglicht einen Wechsel der Schwungphasensteuerungseinrichtung unabhängig von den jeweiligen anderen, mit dem Knieabschnitt verbundenen Komponenten. Es ist also der zur Auswahl der richtigen Schwungphasensteuerungseinrichtung notwendige Austausch leicht möglich. Ebenso lassen sich das Unterschenkelteil mit dem Fußteil oder das Oberschenkelteil, unabhängig von den anderen Komponenten, mit dem Knieabschnitt bzw. mit dem unteren oder dem oberen Knieteil verbinden. Daraus folgt ebenfalls ein leichter Austausch der entsprechenden Komponenten, unabhängig von den anderen bei der Testprothese verwendeten Komponenten. Die Verstelleinrichtung der Verbindungseinheit ermöglicht eine Einstellung der Position des Oberschenkelteils relativ zu dem oberen Knieteil, und daher eine Anpassung der Position des Oberschenkelteils relativ zu den übrigen verwendeten Komponenten, wenn dies aufgrund eines Wechsels der anderen Komponenten notwendig ist.

Weitere Merkmale und Zweckmäßigkeiten der Erfindung ergeben sich aus der Beschreibung von Ausführungsbeispielen anhand der Figuren. Von den Figuren zeigen:
- Fig. 1: eine schematische Darstellung verschiedener Komponenten der Testprothese;
- Fig. 2: eine andere Darstellung verschiedener Komponenten einer anderen Ausführungsform der Testprothese; und
- Fig. 3: eine Explosionsdarstellung einer Ausführungsform einer Verstelleinrichtung.

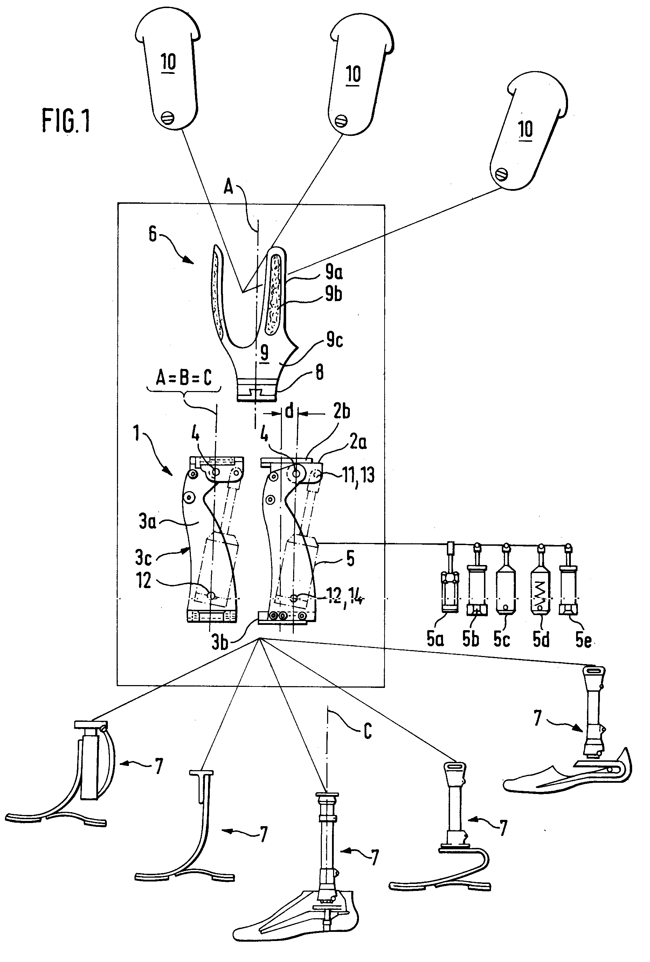

Fig. 1 zeigt verschiedene Komponenten, die in einer Testprothese nach einer Ausführungsform kombiniert werden können. Ein Knieabschnitt 1 weist einen oberen Knieteil 2a, 2b und einen unteren Knieteil 3a, 3b und ein Gelenk 4 auf. Das obere Knieteil weist ein Anschlußteil 2a mit einem ersten Widerlager bzw. Widerlagerpunkt 11 und einer Schlittenführung und einen Schlitten 2b, der mit dem Anschlußteil 2a in der Schlittenführung verschiebbar und einstellbar fest verbunden ist, auf. Das untere Knieteil weist zwei Seitenteile 3a auf, die durch entsprechende Verbindungen und an ihren bodenendigen Seiten durch ein Teil mit einer Schlittenführung rahmenartig verbunden sind. Mit einem so gebildeten Rahmen 3c ist über die Schlittenführung ein Schlitten 3b verschiebbar und einstellbar fest verbunden. Der Rahmen 3c weist ein zweites Widerlager bzw. einen zweiten Widerlagerpunkt 12 auf. Eine Schwungphasensteuerung 5, die als eine Kolben-Zylinder-Einrichtung ausgebildet ist, ist mit ihrer Kolbenstange, die ein erstes Widerlager 13 aufweist, mit dem ersten Widerlagerpunkt 11 des Anschlußteils 2a verbindbar. Die Schwungphasensteuerung 5 weist an ihrem zylinderartigen Ende ein zweites Widerlager 14 auf, mit dem sie mit dem zweiten Widerlagerpunkt 12 des Rahmens 3c verbindbar ist. Bei der in Fig. 1 dargestellten Ausführungsform sind die Widerlager 11, 12, 13, 14 als Durchgangsbohrungen ausgebildet, so daß die jeweiligen ersten Widerlager 11, 13 und die jeweiligen zweiten Widerlager 12, 14 entsprechend durch Schrauben o.ä. verbindbar sind. Auf diese Art können als Schwungphasensteuerungseinrichtung 5 verschiedene Arten von Schwungphasensteuerungseinrichtungen und/oder Standphasensicherungseinrichtungen 5a bis 5e einfach in den Knieabschnitt 1 eingesetzt werden. Diese können servopneumatische, servohydraulische, mechanische o.ä. Kolbenzylinder-Einrichtungen oder andere Schwungphasensteuerungseinrichtungen und Standphasensicherungen sein.

Im oberen Teil von Fig. 1 ist ein Oberschenkelschaftanschluß 9 mit drei schematisch dargestellten Oberschenkelschäften 10 gezeigt. Der Oberschenkelschaftanschluß kann aus einem Kunststoff o.ä. ausgebildet sein und weist bei der dargestellten Ausführungsform drei nach oben weisende Finger 9a, die in einem trichterförmigen unteren Teil 9c zusammenlaufen, auf. In der Ansicht aus Fig. 1 sind nur zwei der Finger zu sehen. Der trichterförmige untere Teil des Oberschenkelschaftanschlusses 9 ist mit einer Verbindungseinheit 8 verbunden. Die Verbindungseinheit 8 ist austauschbar und wird später beschrieben. Über die Verbindungseinheit 8 wird der Oberschenkelschaftanschluß mit dem oberen Knieteil 2, genauer mit dem Schlitten 2b verbunden. Die nach oben weisenden Finger 9a des Oberschenkelschaftanschlusses 9 weisen auf ihrer Innenseite jeweils Klettbänder 9b auf. Diese Klettbänder sind in Fig. 1 schematisch angedeutet. Ein Oberschenkelschaft 10, der an seiner Außenfläche ebenfalls Klettbänder zum Zusammenwirken auf den oben genannten Klettbändern aufweist, wird zwischen die nach oben weisenden Finger des Oberschenkelschaftanschlusses 9 eingeführt, und dann entsprechend durch ein Klebeband, das außen um die Finger des Oberschenkelschaftanschlusses gebunden bzw. gelegt wird, befestigt und gehalten.

Im unteren Teil von Fig. 1 sind fünf verschiedene Unterschenkelteile 7 mit Fußteilen (Unterschenkel-Fußteile) dargestellt. Diese Unterschenkelteile 7 werden beim Ausprobieren bzw. Auswählen entsprechend mit dem Schlitten 3b des unteren Knieteils 3 verbunden.

Wie aus den obigen Ausführungen klar hervorgeht, ist jedes der Teile, d.h. die Schwungphasensicherung, das Oberschenkelteil oder das Unterschenkelteil, separat und unabhängig voneinander austauschbar.

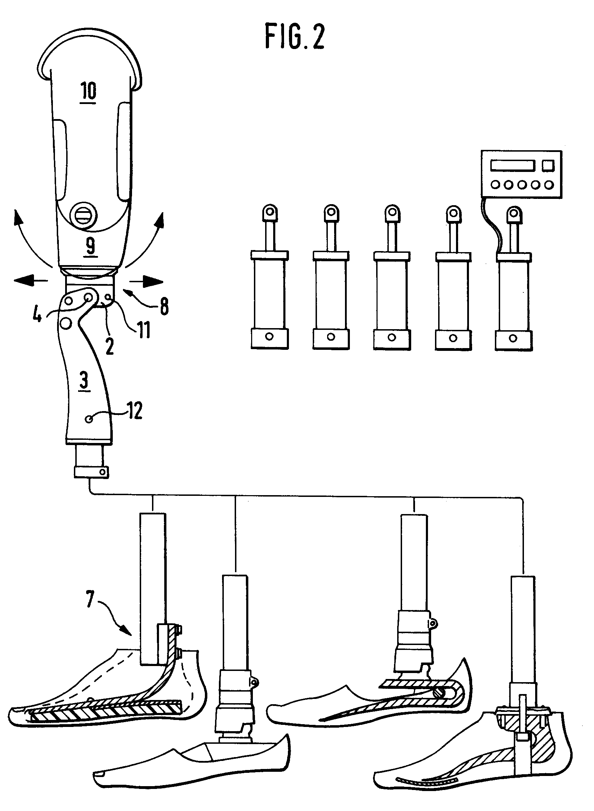

In Fig. 1 ist deutlich zu erkennen, daß die Unterschenkelteile 7 zum Teil deutlich unterschiedlich ausgebildet sind. Die Unterschenkelteile und die Fußteile werden z.B. jeweils nach dem Aktivitätsgrad der Amputierten, der z.B. bei einem Sportler und bei einem wenig aktiven älteren Menschen deutlich unterschiedliche Anforderungen an die Prothese ergibt, ausgewählt. In Fig. 2 sind vier Unterschenkelteile 7 dargestellt, die jeweils rohrförmig ausgebildet sind. Wenn der Unterschied der Unterschenkelteile auf die unterschiedlichen damit verbundenen Fußteile beschränkt ist, so kann ein in Fig. 2 schematisch dargestellter Flansch zur noch einfacheren und schnelleren Verbindung mit dem Knieabschnitt verwendet werden.

Die in den Fig. 1 und 2 dargestellte Verbindungseinheit 8 weist eine Verstelleinrichtung auf. Mit der Verstelleinrichtung kann die Position des Oberschenkelteils 6 mit dem Oberschenkelschaftanschluß 9 und dem Oberschenkelschaft 10 relativ zu dem oberen Knieteil 2 verändert werden. Das bedeutet, daß das Oberschenkelteil 6 relativ zu der Achse des Beines (Beinachse), die die gedachte Linie durch Unterschenkelteil, Knieabschnitt und Oberschenkelteil darstellt, beweglich ist. In Fig. 2 ist durch die Pfeile schematisch eine Verschiebung in einer Ebene parallel und in einer Richtung senkrecht zu der Achse des Gelenkes 4 und eine Verschwenkung in einer Ebene senkrecht zu der Achse des Gelenkes 4 angedeutet, d.h. jeweils in einer zu der Beinachse geneigten Richtung.

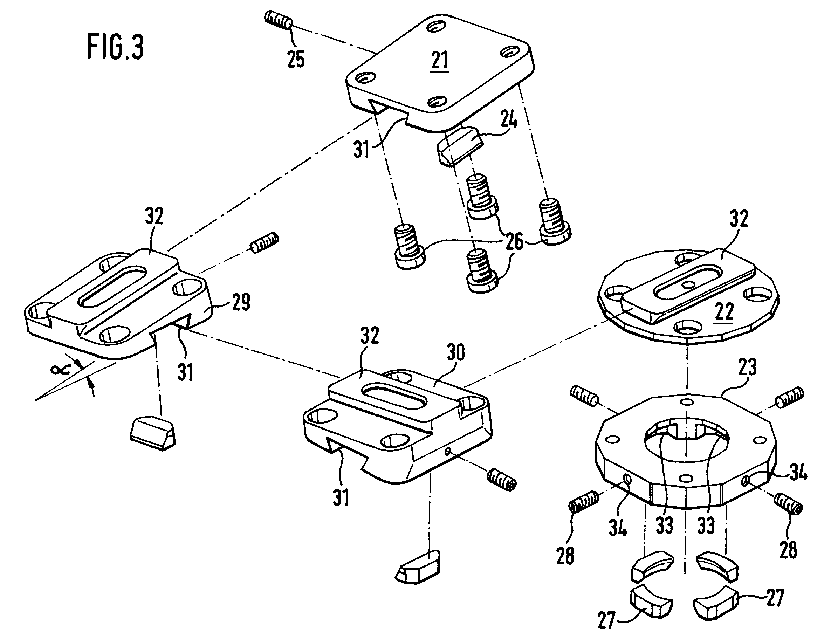

Fig. 3 zeigt eine Ausführungsform einer solchen Verstelleinrichtung der Verbindungseinheit 8 in Explosionsdarstellung. In Fig. 3 sind ein erster Schlittenteil 21, ein zweiter Schlittenteil 22 und ein Drehlagerteil 23 einer Verbindungseinrichtung gezeigt. Das erste Schlittenteil 21 ist plattenförmig mit einer ebenen Oberseite und einer ebenen Unterseite, in der eine Schlittenführungsnut 31 mit einem im wesentlichen trapezförmigen Querschnitt ausgebildet ist. Die trapezförmige Schlittenführungsnut 31 ist so ausgebildet, daß die längere Seite des Trapezes in Richtung der Oberseite liegt. Das zweite Schlittenteil 22 ist ebenfalls im wesentlichen plattenförmig mit einer ebenen Unterseite und einer im wesentlichen ebenen Oberseite, auf der vorspringend ein Schlittenvorsprung 32 mit einem der Schlittenführungsnut 31 entsprechenden Querschnitt ausgebildet ist. Der trapezförmige Querschnitt des Schlittenvorsprungs 32 ist gegenüber dem trapezförmigen Querschnitt der Schlittenführungsnut 31 so gewählt, daß der Schlittenvorsprung in die Schlittenführungsnut 31 einführbar und derart verschiebbar ist, daß die Unterseite des ersten Schlittenteils 21 und die obere Seite des zweiten Schlittenteils 22 in einer Ebene gegeneinander verschiebbar sind. Das erste Schlittenteil weist an seiner Unterseite eine Ausnehmung auf, die so ausgebildet ist, daß ein Klemmkeil 24 in diese Ausnehmung einsetzbar ist. Parallel zu der Oberseite des ersten Schlittenteils 21 ist eine Gewindebohrung in dem ersten Schlittenteil 21 so ausgebildet, daß sie die Ausnehmung für den Klemmkeil 24 erreicht. Eine Schraube 25 wird in die Gewindebohrung eingesetzt, so daß der in die Ausnehmung eingesetzte Klemmkeil 24 durch Drehen der Schraube entsprechend in die Schlittenführungsnut 31 geschoben werden kann. Dadurch kann die Position des ersten Schlittenteils 21 und des zweiten Schlittenteils 22 durch Drehen der Schraube 25 in die Gewindebohrung und durch entsprechendes Klemmen mit dem Klemmkeil 24 fest eingestellt werden. Derart wird durch das erste Schlittenteil 21, das zweite Schlittenteil 22, dem Klemmkeil 24 und die Schraube 25 eine Verstelleinrichtung ausgebildet, die eine Verschiebung in einer Richtung in der Ebene, die parallel zu den Ober- und Unterseiten der Schlittenteile 21, 22 ist, ermöglicht.

Bei der in Fig. 3 gezeigten Ausführungsform ist das erste Schlittenteil 21 der obere Teil der Verbindungseinrichtung 8. Der Oberschenkelschaftanschluß 9 wird durch die in Fig. 3 dargestellten Schrauben 26 mit dem ersten Schlittenteil 21 verbunden. Der zweite Schlittenteil 22 wird mit dem Drehlager 23 durch nicht dargestellte Befestigungselemente verbunden. Das Drehlager 23 ist im wesentlichen plattenförmig ausgebildet. Das Drehlager 23 weist in seinem Zentrum eine im wesentlichen kreisförmige Öffnung auf. In der kreisförmigen Öffnung sind vier symmetrisch angeordnete Ausnehmungen 33 ausgebildet. In diese Ausnehmungen 33 sind entsprechende Klemmkeile 27 einsetzbar. Diese Klemmkeile 27 können durch Schrauben 28, die in eine Gewindebohrung 34 eingesetzt werden, analog zu der Verschiebung des Klemmkeils 24 durch die Schraube 25, in Richtung des Inneren der Öffnung des Drehlagers 23 verschoben werden. Das Drehlager 23 wird mit seiner Öffnung auf einen nicht dargestellten Drehzapfen, der als ein erster Abschnitt der Verbindungseinheit 8 mit dem oberen Knieteil 2 fest verbunden ist, aufgesetzt. Das Drehlager 23 ist als ein zweiter Abschnitt der Verbindungseinheit 8 derart um die Achse des Drehzapfens drehbar, und die Position des Drehlagers 23 relativ zu dem Drehzapfen und damit relativ zu dem oberen Knieteil 2 wird durch Eindrehen der Schrauben 28 in die Gewindebohrungen 34 fest eingestellt.

Derart wird durch Verwendung des ersten und des zweiten Schlittenteils 21, 22, des Drehlagers 23, der Klemmkeile 24, 27 und der Schrauben 25, 28 eine Verbindungseinrichtung mit einer Verstelleinrichtung ausgebildet, die eine Einstellung der Position des Oberschenkelteils 6 relativ zu dem oberen Knieteil 2 in zwei Freiheitsgraden, nämlich der Verschiebung des Schlittens und der Drehung des Drehlagers um den Drehzapfen, erlaubt.

In Fig. 3 sind weiter ein drittes Schlittenteil 29 und ein viertes Schlittenteil 30 dargestellt. Diese Schlittenteile sind im wesentlichen keilförmig ausgebildet, d.h. ihre im wesentlichen ebenen Ober- und Unterseiten sind nicht parallel zueinander, sondern sie weisen einen Winkel α auf, der bevorzugterweise im Bereich von 0-10 Grad liegt. Das dritte und das vierte Schlittenteil 29, 30 weisen auf ihrer Oberseite jeweils einen Schlittenvorsprung 32 und auf ihrer Unterseite eine Schlittenführungsnut 31, die den entsprechenden Vorsprüngen 32 und Nuten 31 der ersten und zweiten Schlittenteile 21, 22 entsprechen, auf. Durch die Verwendung dieser keilförmigen Schlittenteile sind, wenn diese keilförmigen Schlittenteile zwischen das erste Schlittenteil 21 und das zweite Schlittenteil 22 eingesetzt werden, weitere Freiheitsgrade der Verschiebung und zusätzlich durch die Wahl des Winkels α des Keils eine Neigung des ersten Schlittenteils 21 gegenüber dem zweiten Schlittenteil 22 möglich. Dadurch wird als Folge auch das Oberschenkelteil 6 gegenüber dem oberen Knieteil 2 geneigt. Durch die Wahl verschiedener Winkel α für die Keile kann diese Neigung geändert werden. So ist bei der in Fig. 2 dargestellten Ausführungsform die angedeutete Schwenkbewegung bzw. die Änderung der Neigung des Oberschenkelteils gegenüber dem oberen Knieteil möglich.

Alternativ können anstelle des dritten und/oder des vierten keilförmigen Schlittenteils 29, 30 auch ähnliche Schlittenteile mit Nut und Vorsprung verwendet werden, bei denen die Ober- und die Unterseite parallel zueinander verlaufen, d.h. die keinen Winkel relativ zueinander aufweisen.

Unter Bezugnahme auf Fig. 1 wird ein weiteres Merkmal der in Fig. 1 gezeigten Ausführungsform der Testprothese beschrieben. Eine Schwungphasensteuerungseinrichtung 5 weist nicht notwendigerweise auch eine Standphasensicherung, d.h. eine Sicherung gegen das Wegknicken der Prothese im Stand, auf. Daraus folgt, daß der achsgerechte Aufbau einer Testprothese von der Verwendung einer Schwungphasensicherung mit oder ohne Standphasensicherung abhängig ist. Ein achsgerechter Aufbau bedeutet, daß bei einer Schwungphasensteuerung mit Standphasensicherung die Längsachse A des Oberschenkelteils, die Längsachse B des Knieabschnittes, die durch die Achse des Gelenkes 4 und den unteren Anschluß 12 bestimmt wird, und die Längsachse C des Unterschenkelteils 7 miteinander fluchten, wie in Fig. 1 in der linken Darstellung des Knieabschnittes angedeutet, oder daß der Fußpunkt der Achse A auf dem oberen Knieteil 2 mindestens auf der Achse B liegt.

Wenn die Schwungphasensicherung keine Standphasensicherung aufweist, würde die obige fluchtende Ausrichtung ein labiles Gleichgewicht ergeben, und es ist daher für einen sicheren Stand des Oberschenkelamputierten notwendig, daß die Längsachse B des Knieabschnittes, d.h. die Linie, die durch die Schwenkachse des Gelenkes 4 und den Anschluß 12 läuft, gegenüber den Fußpunkten der Achsen A, B des Oberschenkel- und des Unterschenkelteils auf dem oberen und dem unteren Knieteil 2, 3 um einen Abstand d nach hinten gerückt ist. Dieser Zustand ist in Fig. 1 in der rechten Darstellung des Knieabschnittes angedeutet.

Diese Möglichkeit der Verschiebung für einen achsgerechten Aufbau, abhängig von der Verwendung einer Schwungphasensteuerung mit oder ohne Standphasensicherung, wird durch die Schlitten 2b, 3b des oberen Knieteils und des unteren Knieteils 2, 3 ermöglicht.

## Patentansprüche

1. Testprothese mit
einem Oberschenkelteil (6),
einem Unterschenkelteil (7),
einem Knieabschnitt (1), der ein durch ein Gelenk (4) verbundenes oberes Knieteil (2a) und ein unteres Knieteil (3a) aufweist,
einer Schwungphasensteuereinrichtung (5), die mit ihrem einen Ende mit dem oberen Knieteil (2a) und die mit ihrem anderen Ende mit dem unteren Knieteil (3a) verbunden ist, wobei das Oberschenkelteil (6) mit dem oberen Knieteil (2a) und das Unterschenkelteil (7) mit dem unteren Knieteil (3a) verbunden ist,
dadurch gekennzeichnet, daß
das obere Knieteil (2a) mit dem Oberschenkelteil bzw. das untere Knieteil (3a) mit dem Unterschenkelteil (7) durch eine Verbindungseinheit (8), die eine Relativbewegung zwischen den verbundenen Teilen in einer zur Beinachse geneigten Richtung ermöglicht, verbunden ist
und daß eine den Knieabschnitt (1) und das Unterschenkelteil (7) verbindende Schnellwechselvorrichtung vorgesehen ist.

2. Testprothese nach Anspruch 1, dadurch gekennzeichnet, daß das Oberschenkelteil (6) einen die Verbindungseinheit (8) umfassenden Oberschenkelschaftanschluß (9) und einen Oberschenkelschaft (10) aufweist.

3. Testprothese nach Anspruch 1 oder 2, dadurch gekennzeichnet, daß die Verbindungseinheit (8) einen Schlitten mit einem ersten Schlittenteil (21) und einem zweiten Schlittenteil (22) aufweist, die gegeneinander in einer ersten Richtung arretierbar und verschiebbar sind.

4. Testprothese nach einem der Ansprüche 1 bis 3, dadurch gekennzeichnet, daß die Verbindungseinheit (8) einen ersten Abschnitt und einen dazu um eine im wesentlichen zur Beinachse parallelen Achse schwenkbaren zweiten Abschnitt (23) aufweist.

5. Testprothese nach Anspruch 3 oder 4, dadurch gekennzeichnet, daß der Schlitten ein Kreuzschlitten mit einem dritten Schlittenteil (29, 30) ist, wobei das dritte Schlittenteil zwischen das erste Schlittenteil (21) und das zweite Schlittenteil (22) eingesetzt wird, und daß das erste Schlittenteil und das dritte Schlittenteil in der ersten Richtung in einer ersten Ebene und das dritte Schlittenteil und das zweite Schlittenteil in der zweiten Richtung in einer zweiten Ebene gegeneinander arretierbar und verschiebbar sind.

6. Testprothese nach Anspruch 5, dadurch gekennzeichnet, daß bei wenigstens einem der Schlittenteile die Ober- und Unterseiten desselben gegeneinander geneigt sind.

7. Testprothese nach einem der Ansprüche 1 bis 6, dadurch gekennzeichnet, daß der Knieabschnitt einen Rahmen (3c) aufweist.

8. Testprothese nach einem der Ansprüche 1 bis 7, dadurch gekennzeichnet, daß die Schwungphasensteuereinrichtung (5) als Kolben-Zylinder-Einrichtung ausgebildet ist und an einem ersten und einem zweiten Widerlager (11, 12) mit dem Knieabschnitt (1) auswechselbar verbunden ist.

9. Testprothese nach Anspruch 8, dadurch gekennzeichnet, daß wenigstens eines der Widerlager (11, 12) in einer zur Beinachse geneigten Richtung relativ zu dem Rahmen (3c) verschiebbar ist.

10. Testprothese nach einem der Ansprüche 1 bis 9, dadurch gekennzeichnet, daß als Unterschenkelteil eine Mehrzahl von verschiedenen Anforderungen entsprechenden Unterschenkel-Fußteilen vorgesehen sind, die gegeneinander auswechselbar sind.

11. Testprothese nach einem der Ansprüche 1 bis 10, dadurch gekennzeichnet, daß ein Satz gegeneinander austauschbarer Schwungphasensteuereinrichtungen vorgesehen ist, der wenigstens eine servopneumatische, eine servohydraulische und eine mechanische Einrichtung umfaßt.

## Claims

1. Test prosthesis with
a thigh member (6),
a lower leg member (7),
a knee part (1) comprising an upper knee member (2a) connected through a joint (4) and a lower knee member (3a),
a swing phase control (5) having one end connected to the upper knee member (2a) and another end connected to the lower knee member (3a),
whereby the thigh member (6) is connected to the upper knee member (2a) and the lower leg member (7) is connected to the lower knee member (3a),
characterized in that
the upper knee member (2a) is connected with the thigh member and the lower knee member (3a) is connected with the lower leg member (7), respectively, by a connection unit (8) allowing a relative movement between the connected members in a direction inclined with respect to the leg axis,
and that a quick exchange device connecting the knee part (1) with the lower leg member (7) is provided.

2. Test prosthesis according to claim 1,
characterized in that
the thigh member (6) comprises a thigh shaft connection member (9) including the connection unit (8) and a thigh shaft (10).

3. Test prosthesis according to claim 1 or 2,
characterized in that
the connection unit (8) comprises a slide having a first slide member (21) and a second slide member (22), whereby both slide members can be displaced relative to each other in a first direction and locked.

4. Test prosthesis according to one of claims 1 to 3,
characterized in that
the connection unit (8) has a first portion and a second portion (23) which can be pivoted with respect to the first portion around an axis which is substantially parallel to the leg axis.

5. Test prosthesis according to claim 3 or 4,
characterized in that
the slide is a compound slide having a third slide member (29, 30) which is inserted between the first slide member (21) and the second slide member (22), and
that the first slide member and the third slide member can be displaced relative to each other and locked in the first direction in a first plane and the third slide member and the second slide member can be displaced relative to each other and locked in the second direction in a second plane.

6. Test prosthesis according to claim 5,
characterized in that
the upper and lower sides of at least one of the slide members are inclined relative to each other.

7. Test prosthesis according to any of the claims 1 to 6,
characterized in that
the knee portion comprises a frame (3c) and a swing phase control device (5).

8. Test prosthesis according to one of claims 1 to 7,
characterized in that
the swing phase control device (5) is formed as a piston-cylinder-device and replaceably connected to the knee portion (1) at a first and a second abutment (13, 14).

9. Test prosthesis according to claim 8,
characterized in that
at least one of the abutments (11, 12) is displaceable relative to the frame (3c) in an inclined direction relative to the leg axis.

10. Test prosthesis according to any of the claims 1 to 9,
characterized in that
the lower leg member comprises a plurality of interchangeable lower leg-foot members meeting different requirements.

11. Test prosthesis according to any of the claims 1 to 10,
characterized in that
a set of interchangeable swing phase controls is provided, comprising at least one servopneumatic, one servohydraulic and one mechanical device.

## Revendications

1. Prothèse d'essai comprenant :
une pièce pour la cuisse (6),
une pièce pour la jambe (7),
une partie pour le genou (1), qui comporte une pièce de genou supérieure (2a) et une pièce de genou inférieure (3a) reliées par une articulation (4),
un dispositif de commande de la phase de flexion (5), dont une extrémité est assemblée à la pièce de genou supérieure (2a) et l'autre extrémité à la pièce de genou inférieure (3a),
la pièce pour la cuisse (6) étant reliée à la pièce de genou supérieure (2a) et la pièce pour la jambe (7) étant reliée à la pièce de genou inférieure (3a),
caractérisée
en ce que la pièce de genou supérieure (2a) est assemblée avec la pièce pour la cuisse (6), ou la pièce de genou inférieure (3a) avec la pièce pour la jambe (7), par un dispositif de liaison (8), qui autorise un mouvement relatif entre les parties assemblées dans une direction inclinée par rapport à l'axe du membre inférieur,
et en ce qu'il est prévu un dispositif de remplacement rapide pour assembler la partie pour le genou (1) avec la pièce pour la jambe (7).

2. Prothèse d'essai selon la revendication 1, caractérisée en ce que la pièce pour la cuisse (6) est munie d'un dispositif d'assemblage pour tige de cuisse (9), qui comprend le dispositif de liaison (8), et une tige de cuisse (10).

3. Prothèse d'essai selon la revendication 1 ou 2, caractérisée en ce que le dispositif de liaison (8) comprend un chariot avec une première partie de chariot (21) et une deuxième partie de chariot (22), qui peuvent se déplacer dans une première direction et peuvent être bloquées l'une contre l'autre.

4. Prothèse d'essai selon l'une quelconque des revendications 1 à 3, caractérisée en ce que le dispositif de liaison (8) comprend une première partie et une deuxième partie (23) susceptible de pivoter autour d'un axe sensiblement parallèle à l'axe du membre inférieur.

5. Prothèse d'essai selon la revendication 3 ou 4, caractérisée en ce que le chariot forme un chariot croisé avec une troisième partie de chariot (29, 30), la troisième partie de chariot étant insérée entre la première partie de chariot (21) et la deuxième partie de chariot (22), et en ce que la première partie de chariot et la troisième partie de chariot peuvent se déplacer dans la première direction dans un premier plan et peuvent être bloquées l'une contre l'autre et la troisième partie de chariot et la deuxième partie de chariot peuvent se déplacer dans la deuxième direction dans un deuxième plan et peuvent être bloquées l'une contre l'autre.

6. Prothèse d'essai selon la revendication 5, caractérisée en ce que la face supérieure et la face inférieure d'au moins une partie de chariot sont inclinées l'une par rapport à l'autre.

7. Prothèse d'essai selon l'une quelconque des revendications 1 à 6, caractérisée en ce que la partie pour le genou comporte un cadre (3c).

8. Prothèse d'essai selon l'une quelconque des revendications 1 à 7, caractérisée en ce que le dispositif de commande de la phase de flexion (5) est conçu comme un système avec cylindre et piston et est relié de manière amovible avec la partie pour le genou (1) au niveau d'un premier et d'un deuxième abouts (11, 12).

9. Prothèse selon la revendication 8, caractérisée en ce qu'au moins l'un des abouts (11, 12) peut se déplacer par rapport au cadre (3c) dans une direction inclinée par rapport à l'axe du membre inférieur.

10. Prothèse d'essai selon l'une quelconque des revendications 1 à 9, caractérisée en ce que la pièce pour la jambe est choisie parmi plusieurs pièces pour la jambe et le pied, qui répondent chacune à des exigences spécifiques et qui sont interchangeables.

11. Prothèse d'essai selon l'une quelconque des revendications 1 à 10, caractérisée en ce qu'il est prévu un ensemble de dispositifs de commande de la phase de flexion, qui sont interchangeables, lequel ensemble comprend au moins une unité servo-pneumatique, une unité servo-hydraulique et une unité mécanique.
